(19) Europäisches Patentamt
European Patent Office
Office européen des brevets

(11) Publication number: 0 334 300 A1

(12) # EUROPEAN PATENT APPLICATION

(21) Application number: 89105058.5

(51) Int. Cl.[4]: A61K 39/395

(22) Date of filing: 21.03.89

(30) Priority: 21.03.88 US 170550

(43) Date of publication of application: 27.09.89 Bulletin 89/39

(84) Designated Contracting States: DE FR GB IT SE

(71) Applicant: NEORX CORPORATION
410 West Harrison Street
Seattle Washington 98119(US)

(72) Inventor: Fer, Mehmet F.
666 West Olympic Place no.303
Seattle Washington 98119(US)
Inventor: Abrams, Paul G.
2125 1st Avenue, no. 1602
Seattle Washington 98121(US)
Inventor: Anderson, David C.
2415 Thorndyke Avenue
Seattle Washington 98199(US)

(74) Representative: Brown, John David et al
FORRESTER & BOEHMERT
Widenmayerstrasse 4/I
D-8000 München 22(DE)

(54) **The use of monoclonal antibodies and conjugates thereof as signals to direct sensitized effector cells to tumor sites.**

(57) Target cells within a human or mammalian host are eradicated by using a target cell-specific monoclonal antibody to implant an engineered antigen on the target cells in vivo, then administering activated T-lymphocytes which have been sensitized in vitro to recognize the engineered antigen. The engineered antigen is relatively strongly immunogenic toward T-lymphocytes, and the sensitized T-cells selectively exhibit cytotoxicity toward the target cells (e.g. cancer cells) to which the engineered antigen is bound in vivo.

EP 0 334 300 A1

# THE USE OF MONOCLONAL ANTIBODIES AND CONJUGATES THEREOF AS SIGNALS TO DIRECT SENSITIZED EFFECTOR CELLS TO TUMOR SITES

## TECHNICAL FIELD

The present invention provides a method by which the immune system can be manipulated to treat cancers. A method for directing cytotoxic effector cells specifically to target tumor cells *in vivo* is provided. The effector cells (which may be T-cells) may be presensitized *in vitro*.

## BACKGROUND ART

Current conventional methods for treating cancer include surgery, radiotherapy, and chemotherapy, with immunotherapy emerging as the fourth major treatment modality. Since chemotherapy, irradiation, and surgery have certain risks and side effects due to their limited specificity to tumor cells, an elusive search for a less toxic and more effective mode of cancer immunotherapy has long been pursued by scientists. However, several problems exist in the clinical setting. First, some tumor antigens may not be sufficiently "immunogenic" to trigger a clinically relevant response. Additionally, the number of activated lymphocytes available or their level of activity may not be sufficient to successfully combat a large tumor burden. These two major limitations are among the problems which have hampered efforts to design effective immunotherapy systems to date.

With the discovery of monoclonal antibody technology (Kohler and Milstein, 1975), it has now become possible to generate antibodies against tumor-associated antigens in large, uniform quantities. Additionally, methods are now available to expand and activate immune effector cells which can exert anti-tumor effects, both in animal models and in humans (M.A. Cheever et al., *Journal of Immunology*, 1981; T.J. Eberlein et al., *Journal of Experimental Medicine*, 1982; S.A. Rosenberg, *New England Journal of Medicine*, 1985). Most of this work has been made possible through the discovery of lymphokines which are messenger molecules that stimulate or otherwise influence immune effector cells. Particularly, Interleukin-2 (IL-2) has been a useful reagent which stimulates the growth and activity of a subpopulation of T-cells in cell cultures, thus making it possible to generate lymphokine-activated killer (LAK) cells with anti-tumor effects (D.A. Morgan et al., *Science*, 1986; E.A. Grimm et al., *Journal of Experimental Medicine*, 1982).

Certain terms and concepts related to this invention require definition as follows:

1. **Antigen or Immunogen** - A substance which is capable, under appropriate conditions, of inducing a specific immune response and of reacting with the products of that response, that is with specific antibody or with specifically sensitized T-lymphocytes, or both.

2. **Antibody** - An immunoglobulin (a type of protein) molecule synthesized by cells of the lymphoid series, that has a specific amino acid sequence by virtue of which it interacts only with the antigen that induced its synthesis, or with antigens closely related to it.

3. **Monoclonal Antibody** - an antibody derived from a single cell clone (currently, most monoclonal antibodies are generated from fusions leading to hybridoma cell formation as described by Kohler and Millstein, but monoclonal antibodies can also be developed through genetic engineering or potentially by other synthetic methods). Monoclonal antibodies can be used as whole antibodies or fragments thereof.

4. **Hapten** - A specific protein-free substance which has a chemical configuration such that it can interact with specific combining groups on an antibody or with the recognition site on a T-lymphocyte but which, unlike antigenic determinants, does not itself elicit an immune response (e.g., a detectable T-cell response or the formation of a detectable amount of antibody). When coupled with a carrier protein, it does elicit the immune response.

5. **Epitope** - An antigenic determinant (a given antigen may have multiple epitopes).

6. **Lymphokine** - Soluble protein mediators released by certain lymphocytes, which in turn can regulate other cell-mediated immune functions, such as lymphocyte transformation, macrophage activation, or cytotoxicity on other cells.

7. **Mitogen** - A substance that induces mitosis and cell transformation, especially lymphocyte transformation.

As opposed to heteroantisera, monoclonal antibodies can be generated in large quantities, and can be available in uniform batches for use in mammals, including humans. Monoclonal antibodies have, in humans, been demonstrated to:

1. Localize in tumor tissues after intravenous injection (R.K. Oldham et al., Journal of Clinical Oncology, 1984).

2. Exhibit biodistribution selectively in tumors with some non-specific uptake in normal tissues (S.M. Larson et al., Journal of Clinical Investigation, 1983; P.G. Abrams et al., in R.A. Reisfeld and S. Sell (Eds.), Monoclonal Antibodies and Cancer Therapy, 1985).

3. Induce tumor regressions in some patients when antibodies capable of enhancing antibody-dependent cellular cytotoxicity (ADCC) are used (A.N. Houghton, Proceedings of the National Academy of Sciences, U.S.A. 1985).

The antibodies mostly used to boost ADCC have been of the IgG-3 subclass. These antibodies have been used as whole molecules rather than fragments, since the ADCC-inducing activity is related primarily to the Fc portion. The ADCC-related tumor regressions have been inconsistent and occur in a minority of the patients treated (A.N. Houghton, ibid). Additionally, the requirement to use whole antibody reduces flexibility in utilizing fragments, which have different biodistribution characteristics and potentially provide certain advantages in tumor localization. Thus, there clearly is a need to develop methods to increase the capability of antibodies to generate ADCC (and subsequent tumor responses) in a more consistent, reproducible way.

Studies utilizing lymphokines to boost ADCC by independently stimulating effector cells in vivo, or by the administration of lymphokines such as IL-2, are currently in progress. However, this is a non-specific process aimed at activating effector cells in general.

Adoptive immunotherapy using autologous effector cells has recently attracted such attention, after the observations that anti-tumor effects occur in humans (S.A. Rosenberg et al., New England Journal of Medicine, 1985 and 1987; W.H. West et al., New England Journal of Medicine, 1987). It is clear that tumor regressions can occur both in animal models and in humans following the infusion of LAK cells followed by IL-2. These cells are first obtained from the donor (patient) by cytapheresis, and cultured in vitro in the presence of IL-2, thus generating activated killer cells. These cells are expanded in tissue cultures and, when given back to the patient, their continued growth in vivo is further propagated by the administration of IL-2. This manipulation has been shown to result in tumor regressions in various systems. Examples of animal tumor models include the murine FBL-3 leukemia (M.A. Cheever, Journal of Immunology, 1981; T.L. Eberlein et al., Journal of Experimental Medicine, 1982), murine EL-4 (G) lymphoma (M.A. Cheever, ibid), and murine meth-A sarcoma (M.J. Berendt et al., Journal of Experimental Medicine, 1980). Human tumors where efficacy has been observed include renal cell carcinoma, malignant melanoma, and occasionally other types (S.A. Rosenberg et al., New England Journal of Medicine, 1986 and 1987; W.H. West et al., New England Journal of Medicine, 1987; J.R. Durant, New England Journal of Medicine, 1987). In this approach, the activation phenomenon for the killer cells is non-specific and consists of in vitro exposure to IL-2 during cell cultures. Since the cytotoxicity of these cells appears to be exerted selectively in the tumor, it is presumed that the immunologic markers expressed naturally on the tumor cells attract the LAK cells to the tumor sites or perhaps that the IL-2 administered following the LAK cell infusions might have certain effects that either enhance the immunogenicity/antigen expression on the tumor or otherwise contribute to the cytotoxicity. There has been no established method to direct the LAK cells specifically to the tumor. However, it has been observed that cells infiltrating the tumors if obtained from the tumor biopsies and expanded in vitro may be substantially more effective in exerting cytotoxicity (S.A. Rosenberg et al., Science, 1986). If obtaining and cloning this cell population were feasible, the efficacy of such adoptive immunotherapy could potentially increase.

In efforts to explore the selectivity of the lymphokine- activated cells, investigators have, in animal models, exposed the lymphocytes in culture to irradiated cells from the experimental tumor along with IL-2, thereby attempting an in vitro immunization. (S. Gillis, Journal of Experimental Medicine, 1978; M.A. Cheever, Journal of Immunology, 1981). When the expanded effector cell population was tested for cytotoxicity both in in vitro experiments and in vivo for therapy of tumor- bearing animals, they were effective specifically against the immunizing tumor and not against the other tumors used as controls. Similar experiments by others have confirmed this phenomenon (J.L. Strausser et al., Journal of Immunology, 1981; T.C. Eberlein et al., Journal of the National Cancer Institute, 1982). These effector cells have a T-cell phenotype. Thus, it appears that a clone of committed cytotoxic T-cells can be generated by early exposure to tumor antigen in vitro, and these cells may selectively exert their effects on the specific tumor with which they are immunized and not other neoplasms. The application of this technique to human tumors may pose difficulties, since tumor cells or antigens from the given patient may not be accessible in sufficient quantities or may not be sufficiently immunogenic. There may be hazards in administering therapy which might potentially result in the re-entry into the patient of tumor cells that were previously obtained

and used for in vitro immunization of effector cells. In addition, some tumor antigens may not be sufficiently "immunogenic" to trigger a clinically relevant response. Additionally, the number of activated effector cells available or their level of activity may not be sufficient to successfully combat a large tumor burden.

Thus, the various approaches to immunotherapy described above have met with such problems as inconsistency in results, lack of wide clinical applicability in humans, insufficient immunogenicity of some types of tumors, and an inability to generate effector cells of sufficient activity and/or quantity for effective treatment. A need for a method of immunotherapy which circumvents such difficulties remains.

## SUMMARY OF THE INVENTION

The present invention provides a method of eradicating target cells within a human or mammalian host, comprising the steps of:

a) extracting effector cells (or precursors thereof) from the human or mammalian host;

b) cultivating the extracted effector cells in vitro with both an effector cell growth factor and a first immunogenic molecule, thereby producing sensitized effector cells;

c) administering to the host a second immunogenic molecule comprising an antibody or antibody fragment which binds to the target cells, and having at least one moiety in common with the first immunogenic molecule; and

d) administering the sensitized effector cells produced in step (b) to the host.

Preferably, the effector cell growth factor additionally is administered to the host. The effector cell growth factor may be a lymphokine such as interleukin-2 (IL-2). In one embodiment of the invention, administration of the effector cell growth factor to the host begins after Step (c) and continues at least until the completion of Step (d). Alternatively, initiation of administration of effector cell growth factor to the patient may begin at another time, such as after step (d).

The step of extracting effector cells from the host may comprise subjecting the host to one or more daily leukapheresis procedures (i.e., one leukapheresis procedure per 24-hour period for one or more days). In one embodiment of the invention, the effector cells are cytotoxic T-cells. The effector cells are incubated in vitro with both an effector cell growth factor (e.g. a lymphokine such as IL-2) and the first immunogenic molecule to produce sensitized effector cells. The sensitized effector cells recognize the first immunogenic molecule or a moiety thereof.

The first immunogenic molecule may be the same as, or different from, the second immunogenic molecule, but the two immunogenic molecules have at least one moiety in common. The second immunogenic molecule comprises an antibody which binds to the desired target cells, and therefore localizes on the target cells in vivo after administration to the host. The first and second immunogenic molecules are chosen such that effector cells sensitized in vitro in Step (b) to recognize the first immunogenic molecule also recognize the second immunogenic molecule (or the common moiety thereof) localized on the target cells in vivo. The cytotoxic effect of the sensitized effector cells thus is specifically directed toward the target cells in the host.

Mixtures (cocktails) of more than one type of immunogenic molecule may be used in steps (b) and (c) above. Thus, effector cell populations sensitized to recognize different immunogens may be produced, and more than one "engineered antigen" may be implanted on target cells (possibly employing two or more different target-specific antibodies). The use of cocktails may be advantageous when the target tumor comprises heterogeneous tumor cell populations, or to ensure that at least one of the engineered antigens (e.g., histocompatibility antigens) is non-self in the intended patient.

The target cells are undesirable cells to be eradicated within the host, such as cancer cells. When the target cells are cancer cells, and antibody moiety of the second immunogenic molecule (and of the first immunogenic molecule in certain embodiments of the invention) may be a monoclonal antibody which binds to the target cancer cells.

An alternative method for eradicating target cells within a human or mammalian host comprises administering to the host an immunogenic molecule comprising an antibody-immunogen conjugate which binds to the target cells in vivo, wherein binding of the immunogenic molecule to the target cells within the host induces a cytotoxic effector cell response directed at said target cells. Thus, leukapheresis and in vitro sensitization may not be necessary in some cases when the immunogenic molecule is capable of inducing a therapeutically effective cytotoxic effector cell response in vivo without the presensitizing step.

Also provided by the present invention are various immunoconjugates comprising an antibody which binds to a desired target site within a human or mammalian host, and a polypeptide which is foreign to the

host. The polypeptide is bound to the antibody directly or through a linker or spacer molecule. Such immunoconjugates include those in which a Class I or II histocompatibility antigen (non-self or non-human), or a fragment thereof, is bound to a target-specific antibody. The Class I or II histocompatibility antigen fragment may be an alloantigen domain. Peptides which are cytotoxic T-cell epitopes also may be attached to a target-specific antibody to form immunoconjugates of the invention.

## DETAILED DESCRIPTION OF THE INVENTION

The present invention provides a method for eradication of target cells in vivo. The method involves in vitro sensitization of effector cells (e.g. cytotoxic T-cells or natural killer cells) toward an immunogenic molecule. The immunogenic molecule is then bound to the target cells within a patient by employing antibodies specific for the target cells. The sensitized effector cells then are administered to the patient, and their cytotoxic effect is thereby directed specifically against the target cells, which bear the immunogenic molecule against which the effector cells were sensitized in vitro.

As described in the"Background Art" section above, it is now clear that clones of committed effector cells (usually lymphocytes of T-cell origin) can be generated against certain tumor associated antigens (TAA), by in vitro exposure to the TAA and IL-2. These cytotoxic cells can subsequently cause anti-tumor effects both in vitro and in vivo (S. Gillis et al., Journal of Experimental Medicine, 1978; M.A. Cheever et al., Journal of Immunology, 1981; T.J. Eberlein et al., Journal of the National Cancer Institute, 1982; J.L. Strausser et al., Journal of Immunology, 1981). These immune effector cells are thus programmed to recognize and selectively exert their toxicity on tumor cells bearing the selected antigens. The application of this concept could have a substantial impact on cancer treatment. However, several problems exist in the clinical setting. First, some tumor antigens may not be sufficiently "immunogenic" to trigger a clinically relevant response. Additionally, the number of activated effector cells available or their level of activity may not be sufficient to successfully combat a large tumor burden. The invention described herein is designed to overcome these problems by:

1. Employing target cell-specific monoclonal antibodies which themselves are strongly immunogenic, or ensuring the strong immunogenicity by forming immunoconjugates such as monoclonal antibody-hapten conjugates or immunoconjugates comprising a strongly immunogenic polypeptide attached to a monoclonal antibody. These monoclonal antibodies or conjugates thereof such as antibody-hapten conjugates bind to the target cells and serve as "engineered target antigens," once they localize on the target cells (e.g. tumor cells).

2. Producing large number of activated, sensitized cytotoxic effector cells specific for the target cells of interest by leukapheresing the tumor-bearing subject and from that population expanding a sensitized clone of committed effector cells (LAK cells or committed T-cells, or potentially other effector cells) in vitro, cultured in the presence of the antibody or conjugates thereof. This generally is performed in the presence of IL-2 or other lymphokines which stimulate proliferation of the effector cells.

3. Administering to the patients first the antibody or conjugate thereof, and after the immunoconjugate has localized on the target cells to express the engineered antigen, infusing large numbers of the effector cells sensitized in vitro to recognize and exhibit cytotoxicity to these engineered antigen-bearing target cells.

Thus, the monoclonal antibody will serve as a carrier to implant a strong antigenic signal on the target cells, ensuring that the target cells are sufficiently "immunogenic". The engineered antigen bound to the target cells in vivo attracts the presensitized effector cells to the target cells. This procedure in effect constitutes an amplication of the tumor-associated antigen. The effector cells infused will have been co-cultured with this predetermined antigenic signal, thereby sensitizing the effector cells to what can be described as an engineered tumor antigen. Large numbers of cytotoxic effector cells are produced for infusion to the patients by in vitro expansion, and these cells could be further propagated by the use of lymphokines such as IL-2 administered in vivo.

When a target cell-specific monoclonal antibody is sufficiently immunogenic with respect to sensitization of the effector cells, the antibody alone may be employed as the engineered antigen. Alternatively, strongly immunogenic molecules such as haptens or certain polypeptides may be attached to the antibody, and the resulting immunoconjugate may be used as the engineered antigen. Such immunoconjugates and the preparation thereof are discussed in more detail below.

Target-specific antibodies suitable for use in the present invention include polyclonal or monoclonal antibodies with monoclonal antibodies (MAbs) specific for target cells being preferred. A number of

monoclonal antibodies which bind to a specific type of cell have been developed, including MAbs specific for tumor-associated antigens in humans, and procedures for production of such antibodies are known. Among the many such MAbs which may be used are anti-TAC, or other IL-2 receptor antibodies; 9.2.27 and NR-M1-05 to the 250 kilodalton human melanoma-associated proteoglycan; NR-Lu-10 to 37-40 kilodalton pancarcinoma glycoprotein; and $OVB_3$ to an as yet unidentified cancer-associated antigen. Antibodies derived through genetic engineering or protein engineering may be employed as well. The antibody employed in the present invention may be an intact molecule, a fragment thereof, or a functional equivalent thereof. Examples of antibody fragments are $F(ab')_2$, Fab', Fab, and Fv fragments, which may be produced by conventional methods.

In summary, the method of the present invention provides a procedure for amplifying the antigenicity of target cells such as tumor cells by using monoclonal antibody carriers to implant strong antigenic signals on the tumor cells. In order to enhance the immunocompetency of the host, the ex vivo manipulation of effector cells with in vitro exposure to lymphokines and the predetermined ("engineered") antigenic signal is performed. After the engineered antigen (which comprises a target-cell specific monoclonal antibody) is administered to the patient and is localized on the target cells, the cytotoxic effector cells preconditioned to recognize the antigenic signal are infused, thereby amplifying the immune response to the tumor.

In accordance with the method of the present invention, effector cells are extracted from a human or mammalian host. The effector cells are cells capable of being "sensitized" such that the sensitized effector cells recognize a particular antigen, and have a cytotoxic effect on target cells bearing that antigen. The effector cells used could include what is described as "committed T-cells", along with a variety of cytotoxic cells designated otherwise, such as LAK cells, natural killer (NK) cells, and other leukocytes and macrophages which could potentially be sensitized and manipulated similarly. In one embodiment of the invention, the effector cells are cytotoxic T-lymphocytes, also called cytotoxic T-cells.

The effector cells are extracted from the host (i.e., the patient to be treated) by any suitable procedure for collecting the cells from the patient and separating the desired effector cells from any other cell types or biological materials which may be simultaneously extracted. Conventional cell harvesting or cytapheresis (e.g. leukapheresis) procedures may be used, and the effector cell extraction procedure may be repeated to obtain a larger quantity of effector cells. For example, the host may be subjected to one leukapheresis procedure per day for one or more days (e.g., for five days to collect a large number of effector cells such as T-cells).

The extracted effector cells are incubated in vitro with both an effector cell growth factor and a first immunogenic molecule to produce sensitized effector cells. The effector cell growth factor used may vary according to the type of effector cells employed, and may be any growth factor which stimulates the propagation of the cultured cells and enhances the activation of the cells so that sensitized cytotoxic effector cells are produced. Among the suitable growth factors are monokines for monocytes and lymphokines (e.g. IL-2) for lymphocytes such as T-lymphocytes.

The generation of large numbers of lymphokine-activated killer cells for clinical application has been described in detail in the literature (Rosenberg, S.A. et al., New England Journal of Medicine, 1985; Muul, L.M., Journal of Immunologic Methods, 1986; West, W.H. et al., New England Journal of Medicine, 1987). As discussed above, early exposure of effector cells to target antigens in animal models has also been described in depth (Gillis, S. et al., Journal of Experimental Medicine, 1987; Cheever, M.A. et al., Journal of Immunology, 1981). Variations based on these described methods are suitable for use in the method of this invention.

The first immunogenic molecule (with which the effector cells are incubated) may be the same as or different from the second immunogenic molecule (which is administered to the patient), with the provision that the two types of immunogenic molecules have at least one moiety in common such that effector cells sensitized in vitro in the presence of the first immunogenic molecule will recognize the second immunogenic molecule in vivo.

When an antibody (preferably a monoclonal antibody) specific for the target cells of interest is sufficiently immunogenic, the antibody alone may be used as the first and the second immunogenic molecules. The term "immunogenic" as used herein means capable of inducing sensitization of the effector cells; i.e., the effector cells can be sensitized to recognize the molecule.

Certain antibody production methods may result in antibodies having increased immunogenicity. For example, genetically engineered antibodies produced in yeast are glycosylated to incorporate mannose molecules, resulting in mannan structures which are antigenic. These antibodies may be sufficiently immunogenic to be used as the first immunogenic molecule in this invention, without the addition of other structures to the antibody.

Antibody production methods have been described extensively in the prior art and will not be further

discussed here (Kohler and Milstein, 1975; H. Zola (ed.), 1987). Whole antibody or antibody fragments [Fab, Fab′, F(ab′)$_2$, or smaller fragments produced by conventional techniques] may be used as appropriate, provided that these fragments retain immunoreactivity and immunogenicity.

The immunogenicity of a particular antibody may be tested in an appropriate conventional cytotoxicity assay or in animal studies. Such in vitro assays or studies thus may be used to determine whether a particular antibody is sufficiently immunogenic for use as the first immunogenic molecule.

Alternatively, various compounds may be attached to an antibody to increase the immunogenicity thereof. Among the types of compounds which may be attached to antibodies for this purpose are haptens and polypeptides which are "foreign" to the intended patient (such as antigens, mitogens, other foreign proteins, and fragments thereof, or peptides that activate cytotoxic T-cells). In some cases, attachment of the compound to the antibody through a spacer (i.e., a linker molecule which serves to physically separate the compound from the antibody) may increase the immunogenicity.

A definition of haptens is presented above. Hapten groups which may be attached to an antibody in accordance with the present invention include but are not limited to benzoate groups, nitrophenol groups, other small molecules such as acetic acid and derivatives, penicillenic acid and derivatives, sulfanilic acid derivatives, hexoseamines, and perhaps ribonucleotides or ribonucleosides, and isocyanates and isothiocyanates. These represent just a sample of the various molecules which may be used as the hapten moiety for the purposes of this invention, and other hapten groups could be suitable as well. Certain of the larger hapten molecules may be more immunogenic than the smaller haptens and may induce a more effective T-cell response.

The chemical configuration of some of these hapten molecules are as follows:

Benzoic Acid

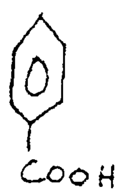

Dinitrochlorobenzene

(DNCB)

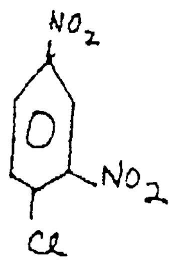

Dinitrofluorobenzene (DNFB)

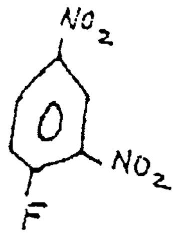

Picrylchloride

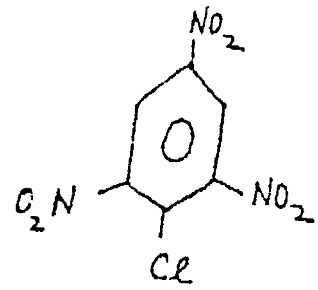

p-aminobenzenearsonate

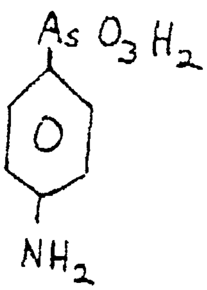

p-azo-benzenearsonate

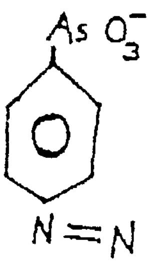

Acetic Acid

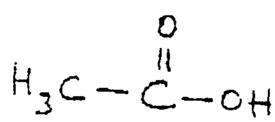

Dinitrophenol

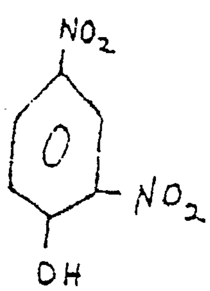

Trinitrophenol

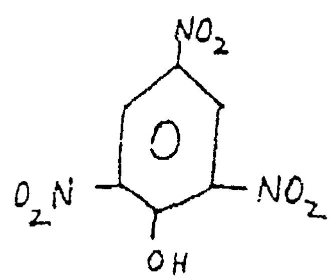

Trinitrophenol - ε - Aminocaproic Acid

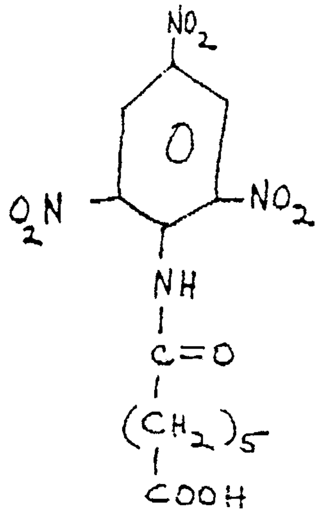

3-Iodo-4-Hydroxyl-5 Nitrophenylacetic Acid

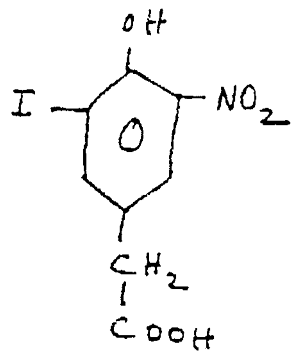

p-Hydroxyphenylacetic Acid

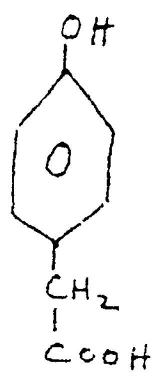

p-Sulfanilic Acid

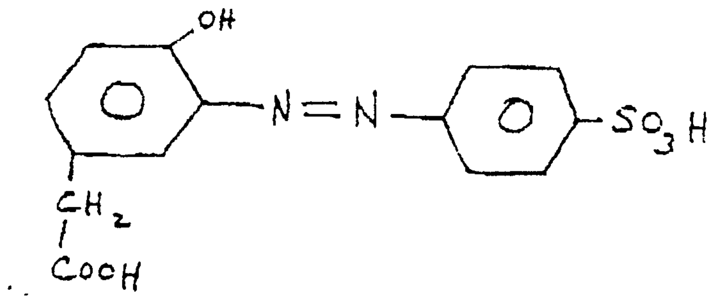

2, 4 Diisocyanate

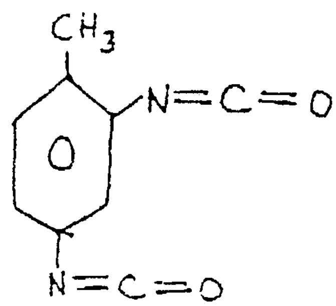

Fluorescein Isothiocyanate

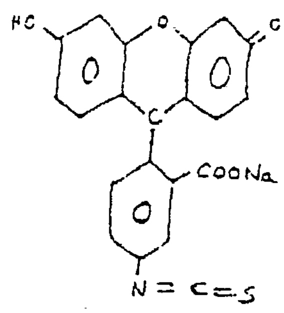

N-Acetyl-Glucosamine

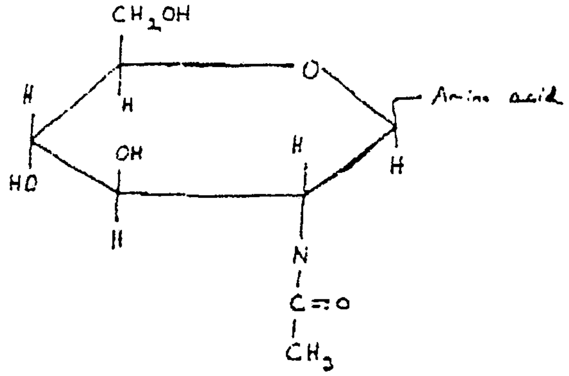

A variety of antigens can also be conjugated to antibodies to enhance the immunogenicity of the antibody molecule. These may include polypeptides which are "foreign" to the intended host; i.e., will be recognized as foreign (non-self) polypeptides by the host's immune system. Examples of such polypeptides for use in a human patient include hydrolysed dextrans, flagellins, fragments of keyhole limpet hemocyanin, histocompatibility antigens, bacterial cell surface proteins, viral coat proteins, and fragments thereof. Smaller molecules (i.e., polypeptide fragments) are generally preferred in efforts to avoid interference with the biodistribution of the antibody (or antibody fragment). Synthetic polypeptides produced through such methods as in vitro polypeptide synthesis, protein engineering, and recombinant DNA technology could also be attacked to an antibody to enhance the antigenicity of the antibody molecule.

T-cell mitogens such as the proteins pokeweed antiviral protein (PAP) and phytohemagglutinin (PHA) also may be used. Of these, PAP may be more suitable because of its lower molecular weight (32,000) as opposed to PHA (140,000).

Polypeptides which do not have analogous counterparts in humans generally may be more immunogenic and therefore preferred for use in the present invention. Such polypeptides include bacterial cell surface proteins, viral coat proteins, and fragments thereof, among others. Several such antigens (to which human T-cells are known to respond) have been characterized. For example, the amino acid sequence of influenza hemagglutinin is known, and this protein (or antigenic fragments thereof) thus may be purified or produced synthetically for use in the present invention.

The effector cells which will be used to kill tumor cells generally fall into two broad classes [Lanier and Phillips Immunology Today 7, 132 (1986)]. First are the natural killer (NK) cells and a subset of cytotoxic T cells, which act on target cells without major histocompatibility complex (MHC) restriction (i.e., which do not need an MHC Class I protein for their activity). The above mentioned antigens attached directly to antibodies are designed to activate these cells. A variety of antigens are proposed to activate natural killer cells since very little is known about their structural requirements or about the cell surface receptors on the NK cell surface to which they bind. The second class includes cytotoxic T cells which are MHC-restricted. These perform a central hole in the immune response to self cells infected by foreign organisms and are thought to be involved in immune system surveillance for tumor cells. However, some malignant cells are thought to escape immune system surveillance due to the lack of immunogenicity of tumor-associated antigens, and to various suppressive mechanisms present in tumor-bearing animals [Berendt MJ and North RF, J. Exp. Med. 151, 69 (1980)]. A number of attempts have been made to increase tumor immunogenicity by coupling antigenic determinants to tumor cells or inducing antigenic variants. [See Flood, PM et al., J. Immunol. 138, 3573-9 (1987) and references therein].

For some tumors such as certain types of leukemia, bladder, sarcoma, or methylcholanthrena-induced tumors, clones derived from the tumor metastasize more readily when they are deficient in MHC antigen expression [Festenstein H and Garrido, F, Nature 322, 502 (1986)]. Other tumors for which lack of MHC antigen expression may correlate with tumor growth may include a mouse lung carcinoma and possibly human small cell lung carcinoma, and other tumors [see Bahler, DW et al., PNAS 84, 4562-6, (1987) and references therein]. However, a number of examples also exist where self MHC antigen expression may enhance tumor growth, such as Maloney virus-induced YAC-I tumor cells, and there seems to be extreme heterogeneity in MHC antigen expression in individual cells found within solid tumors such as breast carcinomas, colorectal carcinomas, or melanomas (Festenstein and Garrido, 1986).

All of these studies have focused on self MHC antigen expression. A novel approach to immune therapy

which may circumvent the lack of MHC antigen expression on some tumors, while possibly avoiding the risk of MHC self antigen presence on the surface of other tumor cells, includes the intentional labeling of tumor cells with immunoconjugates comprising non-self MHC antigens attached to tumor-specific antibodies, in accordance with the present invention. It is known that expression of aberrant MHC-like antigens has resulted in the rejection of Rous sarcoma virus-induced tumors which do not express normal self Class I MHC antigens (Festenstein and Garrido, 1986). Likewise, the adoptive transfer of allogeneic T-cells reactive to the MHC antigens themselves has been used successfully (Meredith RF and Okunewick JP, Transplantation 35, 378-84, 1983). This approach should take advantage of the classical immune system surveillance and rejection of foreign cells, which are recognized by the presence on their surfaces of non-self Class I or II MHC antigens. Such a response may be important, for example, in graft-versus-host disease [R. Storb, "Graft-versus-host disease after marrow transplantation" in Transplantation: Approaches to Graft Rejection - (Meryman HT, ed) Alan R. Liss, New York, 1986] or in transplant rejection in HLA-mismatched individuals.

In one embodiment of the present invention, the extramembranous part of non-self or even non-human MHC Class I or II antigen protein's heavy (alpha) chain (many of whose sequences have been elucidated: see Figueroa F and Klein J, Immunology Today 7, 41-44, 1986 or Giles RC and Capra JD, Advances in Immunology 37, 1-71, 1985, for example) can be conjugated to antibodies directed against tumor-specific antigens, to direct cytotoxic T cells to recognize the tumor cells as "foreign" and lyse them. In another approach, the alloantigen site; i.e., the cytotoxic T cell recognition site on the MHC Class I or II antigen, can be covalently conjugated to anti-tumor antibodies. The alloantigens sites have not been defined precisely in the three-dimensional structure or sequence of the best-studied HLA antigen, HLA-A2 (Bjorkman PJ et al., Nature 329, 506-518, 1987) although fragments of HLA-A2 which inhibit cytotoxic T-lymphocyte (CTL) response have been described (Parham et al., Nature 325, 625, 1987). Thus, either soluble non-self Class I or Class II histocompatibility antigens, human or non-human, or fragments which contain the alloantigenic site, may be conjugated to anti-tumor antibodies. In alternate embodiments of this invention, the immunoconjugate is created as a fusion protein of anti-tumor antibodies or fragments thereof with the soluble part of Class I or II non-self antigens, or alloantigenic fragments of these antigens. Alternatively, the soluble Class I or II non-self MHC antigen (i.e., histocompatibility antigen) or alloantigenic fragment can be created by expression of DNA coding the sequence, or by fragmentation of the natural MHC protein with proteases or organic chemicals.

The use of immunoconjugates, including non-self HLA antigens, may induce activation of helper T cells reactive against the HLA antigens. These cells will augment cytotoxic T cell responses against the immunoconjugate or HLA antigens, and will also assist T cell responses to other cell surface antigens, which will enhance the tumor-killing effect sought here (Fujiwara H et al., in Cellular Immunotherapy of Cancer, R. L. Truitt, R. P. Gale, M. M. Bortin, eds., New York: Alan R. Liss, Inc., pp 335-344, 1987).

Another class of immunogens which can be attached to anti- tumor antibodies include peptide fragments of foreign immunogenic proteins which are cytotoxic T cell epitopes. These peptides have been shown to induce proliferation of antigen-specific cytotoxic T cells, and can be attached either directly to the antibodies or with long spacer arms. Examples of human cytotoxic T cell epitopes from influenza virus nucleoprotein include the peptides SAAFEDLRVLSFIRG and IASNENMDAMESSTLE (Townsend, A.R.M. et al., Cell 44, 959-968, 1986).

The first and second immunogenic molecules may be the same, wherein both molecules are an immunogenic target-specific monoclonal antibody or both molecules are the same immunoconjugate comprising a target-specific monoclonal antibody having one of the above-described haptens of foreign polypeptides attached thereto.

In alternative embodiments of the invention, the first immunogenic molecule need not comprise an antibody. For example, the first immunogenic molecule may be one of the above-described foreign polypeptides (e.g., an antigen, mitogen, or fragments thereof). In this case, the second immunogenic molecule comprises the first immunogenic molecule attached to a monoclonal antibody which binds to the target cells. The effector cells sensitized in vitro to recognize the first immunogenic molecule also recognize the molecule in vivo, and exhibit cytotoxicity toward the target cells, which have the immunogen bound thereto through the monoclonal antibody.

In a second example, the first immunogenic molecule may comprise a hapten bound to one of the above-described polypeptides, in which case the second immunogenic molecule comprises the same hapten attached to a monoclonal antibody which binds to the target cells. the presumption here would be that the effector cells would still recognize the haptenic groups but would perhaps be better immunized when the haptens are on a different protein. Alternately, such manipulations could prove more cost effective or practical (such as the case might be if the hapten is conjugated to albumin and the antibody, the former used in the in vitro procedures and the latter in vivo).

A number of standard chemical linking procedures can be used to attach the various haptens or polypeptides to antibodies. The procedure for attaching a compound to an antibody will vary according to the chemical structure of the compound. Antibodies are proteins which contain a variety of functional groups; e.g., carboxylic acid (COOH) or free amine ($-NH_2$) groups, which are available for reaction with a suitable functional group on the compound to bind the compound thereto. Alternatively, the antibody and/or compound may be derivatized to expose or attach additional reactive functional groups. The derivatization may involve attachment of any of a number of linker molecules such as those available from Pierce Chemical Company, Rockford, Illinois. (See the Pierce 1986-87 General Catalog, pages 313-354). A bifunctional linker having one functional group reactive with a group on a particular agent, and another group reactive with an antibody, may be used to form the desired immunoconjugate.

Alternatively, derivatization may involve chemical treatment of the antibody; e.g., glycol cleavage of the sugar moiety of the glycoprotein antibody with periodate to generate free aldehyde groups. The free aldehyde groups on the antibody may be reacted with free amine or hydrazine groups on a compound to bind the compound thereto. (See U.S. Patent No. 4,671,958). Procedures for generation of free sulfhydryl groups on antibodies or antibody fragments also are known. (See U.S. Patent No. 4,659,839).

Many procedures and linker molecules for attachment of various compounds, including polypeptides, to proteins such as antibodies are known. See for example, U.S. Patents Numbers 4,671,958; 4,659,839, 4,414,148; 4,699,784; 4,680,338; 4,569,789; and 4,590,071.

The linking reaction should be conducted under conditions mild enough to prevent denaturation or otherwise damaging the biological activity of the antibody or polypeptide. The resulting linkage should be stable so that the hapten or polypeptide does not disassociate from the antibody in vivo but remains bound to the target cell (through the antibody) at least long enough to induce a cytotoxic T-cell response to the target cells.

Some of the smaller hapten molecules such as benzoic acid could be easily conjugated to antibodies in the presence of coupling reagents such as ECDI (ethyl dimethylaminopropyl carbodiimide). Other procedures may require linkers such as SMCC (succinimidyl maleimidomethyl cyclohexane carboxylate).

Some examples of how haptens may be linked to the antibody molecules (or fragments) are as follows:

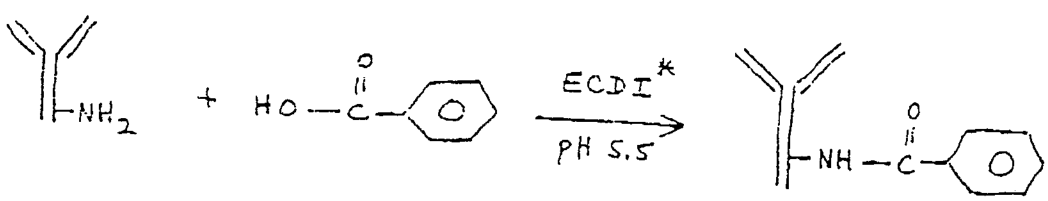


Antibody Benzoic Acid

* ECDI: 1-ethyl-3-(3-dimethylaminopropyl)carbodiimide

Hydroxyphenyl acetic acid, acetic acid, 3-iodo-4 hydroxy-5- nitrophenyl acetic acid, and p-sulfanilic acid can be conjugated to the antibodies by methods similar or comparable to that described above for benzoic acid. Optimal conditions may vary.

Nitrophenyl derivatives are also possible to link with antibodies by interacting with the $\epsilon$- $NH_2$ group of lysine residues, or $\alpha$ $-NH_2$ groups of terminal amino acids, or the SH group of cysteines.

For example:

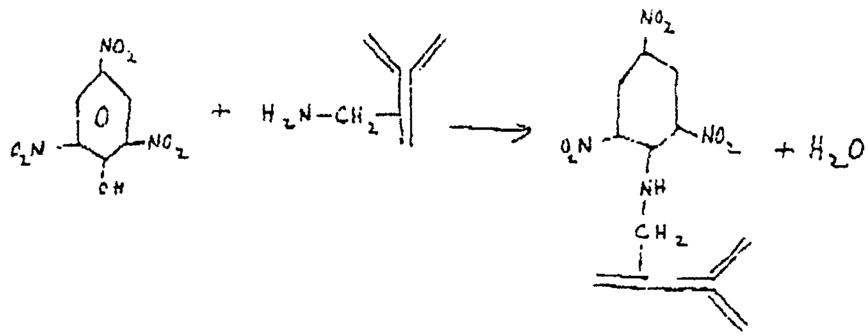

Another example may be as follows:

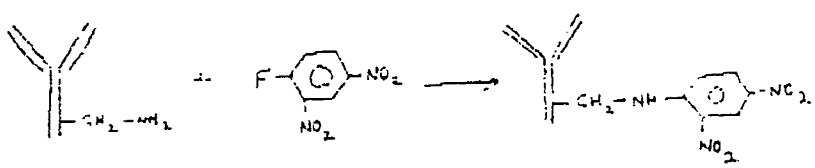

| | | |
|---|---|---|
| Antibody (with $-NH_2$ group) | DNFB | Antibody-DNFB Conjugate |

Another example may be as follows:

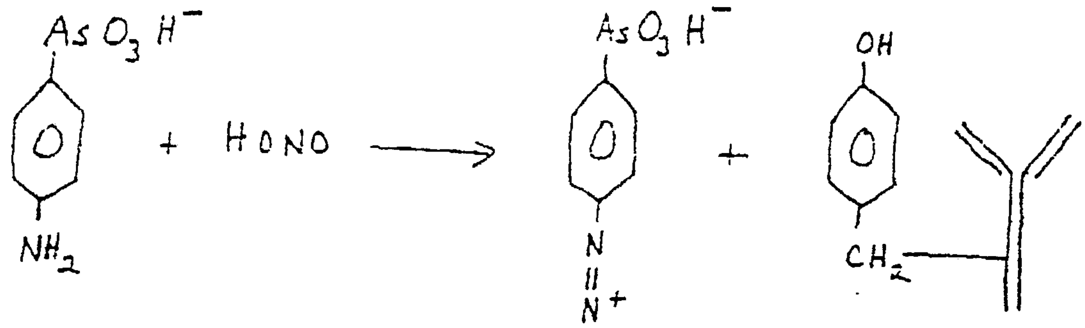

p-aminobenzene arsonate (arsanilic acid)

Diazonium compound

Antibody (with tyro- sine residue)

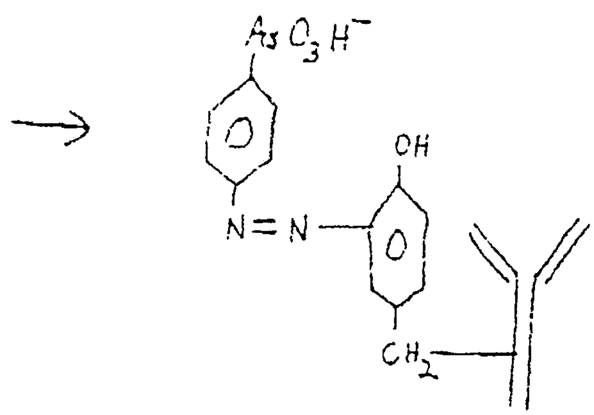

Antibody-p- azobenzene arsonate conjugate

More detailed examples for a linking procedure can be found below in examples 1a and 2a.

Advantageously, an effector cell growth factor is administered to the patient to enhance the propagation and activity of the sensitized effector cells in vivo, Generally, the administered effector cell growth factor is the same growth factor with which the effector cells are incubated in vitro during the sensitization step. Suitable growth factors vary according to the type of effector cells employed, and include lymphokines such as IL-2, as described above.

Procedures for administration of the growth factor may vary. For example, several doses may be given, or infusion may be continued for several days. Examples of suitable procedures are presented in the examples below.

In some cases, the "engineered antigen" to be bound to the target cells (through an antibody) may be

sufficiently immunogenic to induce a therapeutically effective cytotoxic effector cell response in vivo without sensitizing extracted effector cells to an immunogenic molecule in vitro. Therefore, an alternative method for eradicating target cells within a human or mammalian host comprises administering to the host an immunogenic molecule comprising an antibody-immunogen conjugate which binds to the target cells in vivo, wherein binding of the immunogenic molecule to the target cells within the host induces a cytotoxic effector cell response directed at said target cells. The immunogenic molecule comprises one of the above-described foreign polypeptides attached to a target-specific antibody.

Administration of the immunogenic molecule (the engineered antigen) serves to enhance a host's immune response to target cells such as tumor cells by implanting an antigenic signal on the target cells that induces a stronger effector cell response (i.e. is more antigenic) than would the target cells alone. As discussed above, certain types of tumor cells are not sufficiently immunogenic to induce a strong immune response. For example, a variety of types of cancer cells are believed to be deficient in expression of Class I histocompatibility antigens which may be a mechanism by which these cells avoid immune system surveillance.

When certain engineered antigens of sufficient immunogenicity are bound to such target cells (through the antibody), the host's natural effector cell population responds. For example, when the immunogenic molecule comprises a non-self histocompatibility antigen or fragment thereof attached to an antibody (as described above), the host's natural T-cell surveillance is expected to result in a cytotoxic T-cell response directed against the engineered antigen-bearing target cells. A second example is an immunogenic molecule comprising one of the above-described antigenic polypeptides derived from a virus or bacterium, attached to the antibody. Many patients may already have T-cells sensitized toward such polypeptides as a result of previous exposure to the virus or bacterium.

Optionally, the method may comprise the additional stop of extracting effector cells from the host and incubating the extracted effector cells in vitro with a lymphokine to activate them. Procedures for activation of effector cells such as T-cells with lymphokines (e.g. IL-2) to generate lymphokine activated killer cells (LAK cells) have been described above. The activated effector cells then are administered to the patient to whom the immunogenic molecule has been administered. A lymphokine may also be administered to the patient, as described above.

It is now clear that antibodies or fragments administered intravenously can localize in tumor tissues. Generally speaking, this would be the route of delivery. However, there may be variations depending on the clinical setting, such intralymphatic or intracavitary (into the pleura or peritoneum) as appropriate.

It is expected that the greatest therapeutic benefits (relatively speaking) may be seen in patients with more intact immune systems (without interference by recent chemotherapy or radiation therapy). However, the treatment described in the invention can be applied to patients simultaneously or in conjunction with other therapeutic modalities. For example, debulking surgery could be performed prior to treatment, to limit the tumor burden. It is possible that radiation therapy to the bulky tumors simultaneously delivered may also work toward the same goal. In this event, patients would need to be cytapheresed prior to the institution of radiation therapy in order to protect the circulating stem cells. After these effector cells are expanded in culture, radiation could be instituted to be given after the antibody and prior to the infusion of each effector cell dose. In this event, the concept would be that tumor cells damaged by radiation, even if they would eventually prove to be radioresistant, would be potentially more susceptible to cytotoxic effects of the immune effector cells, which would be attracted to the area simply because of their recognition of the "engineered antigen" delivered previously.

Target-specific antibodies chosen for use in the present invention advantageously should not be rapidly internalized by the target cells, since the antibody (or conjugate thereof) should remain on the target cell surface long enough to attract effector cells to the target site. If the antibody or conjugate (the "engineered antigen") is internalized into the tumor cell, the effector cells may not recognize it as a surface marker. On the other hand, the internalization of the antibody would be a desired effect if a drug or toxin is conjugated to it. Thus, it is possible that dual conjugations could be performed to link both the immunogenic groups (e.g. haptens or polypeptides) and drug or toxins simultaneously to the antibody. In this event, whether the antibody remains on the surface or is internalized, the tumor cell would be subjected to toxicity. Techniques for attaching drugs and toxins to antibodies are known, as described above.

The method described herein may also be used in conjunction with administration of other biological response modifiers to the patient. For example, agents that enhance antigen expression by the tumor (such as interferons) may increase the number of available sites for the binding of the "engineered antigen" which would, in effect, amplify the immunogenicity of the tumor cell.

The following examples are provided for purposes of illustration, not for purposes of limitation.

### Example 1a:

In this example, benzoate groups are conjugated to a monoclonal antibody that recognizes a melanoma-associated antigen, for the treatment of a patient with malignant melanoma.

### The Antibody:

The antibody used in this example is murine monoclonal antibody NRX-118.00 which is of the IgG2b subclass. This antibody was developed by fusing mouse splenocytes immunized with melanoma proteins, with a murine myeloma cell line. This antibody recognizes a 250 kilodalton glycoprotein/proteoglycan melanoma-associated antigen. This antibody can be used as a whole molecule or as the Fab or Fab′ fragments.

### The Conjugation Procedure:

To a solution of antibody (2-5 mg/mL) in phosphate buffer (0.01 molar, pH = 5.5) containing 0.1 M, 1-ethyl-3-(3-dimethilaminopropyl)carbodiimide (ECDI) 20 equivalents of benzoic acid (4 mg/mL DMSO solution) added dropwise with vigorous stirring. The reaction mixture is kept at room temperature for two hours. At the end of this period, unreacted benzoic acid and ECDI is removed by a disposable size exclusion column (such as a BioRad PD-10 or comparable column). The prepared antibody is then tested for sterility and safety (using standard tests such as cultures, rabid pyrogen testing, and limulus amebocyte assay, etc.). The antibody solution is then brought to the appropriate concentration for injection and also for use in the *in vitro* immunization procedures.

In the procedure as described, the ECDI catalyzes a reaction between the amino groups in the protein molecule to react with the hydroxyl group in benzoic acid, thus attaching the benzoate group to the antibody. The chemical reaction that occurs is depicted as follows:

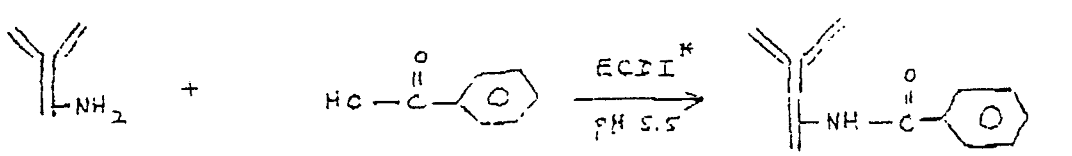


Antibody Benzoic Acid

* ECDI: 1-ethyl-3-(3-dimethylaminopropyl)carbodiimide

This procedure could be performed by using other comparable water-soluble carbodiimides.

### Patient Selection:

The patient is a 29-year-old previously healthy male with malignant melanoma metastatic to multiple subcutaneous nodules. He first undergoes leukopheresis procedures to harvest immune effector cells. After the appropriate expansion is performed *in vitro*, the antibody/conjugate is given, followed by the effector cells, followed by the lymphokine, as follows:

### The Harvesting and Generation of Immune Effector Cells:

The effector cell population will be expanded similarly to what has extensively been described in the literature (Rosenberg, S.A. et al., New England Journal of Medicine, 1985), as follows:

Large numbers of lymphocytes are obtained by daily, repeated pheresis procedures with a continuous flow cell separator. It is targeted that 5 x $10^9$ to 5 x $10^{10}$ mononuclear cells are harvested during each procedure. Acid-citrate-dextrose and heparin are used as anticoagulants in the transfer bag in which the mononuclear cells are collected. The final volume in the leukopheresis bag is 300-400 mL. This procedure is repeated for five days.

The mononuclear cells are separated by a ficoll-hypaque density gradient. Two to three parts of Hanks′ balanced salt solution without calcium or magnesium are mixed with one part of the leukopheresis cell suspension. 40 mL of the diluted cell suspension is poured into 50 mL conical centrifuge tubes and underlayered with 10 mL of lymphocyte separation medium. The cells are then centrifuged at 900 x g for 15 minutes and lymphocytes harvested, washed twice with Hanks′ solution, and resuspended in tissue culture medium which includes RPMI-1640 with 10 units of penicillin per mL, 10 micrograms of streptomycin sulfate per mL, two mmol of glutamine per liter, five micrograms of gentamycin sulfate per mL, and 2% heat inactivated human AB serum. Each liter of cell suspension containing 1 to 1.5 x $10^6$ cells per mL are added to 2.5 liter roller bottles. Into these bottles recombinant Interleukin-2 is added at a final concentration of 1,000 units per mL along with the anti-melanoma monoclonal antibody/hapten conjugate prepared above (the "engineered tumor antigen") at a concentration of 0.01 mg/mL (10 mg into each 1000 cc roller bottle). The roller bottles are incubated at 37 °C with continuous rotation at 0.5 to 1 revolution per minute for three to five days. The cells are then centrifuged at 500 x g for 15 minutes in 1 L bottles, and the pellets pooled in 250 mL centrifuge tubes, washed twice more in Hanks′ balanced salt solution without calcium, magnesium, or phenol red, and placed into infusion medium, which consists of 200 mL of 0.9% sodium chloride and 5% normal human serum albumin with 75,000 units of recombinant Interleukin-2. The cells are then filtered through a sterile nylon blood administration filter (110 mesh) and placed into a transfer pack for administration. Bacterial cultures are obtained in the beginning of the cell cultures and prior to administration to the patient, along with pyrogen testing.

### Administration of Therapy:

The monoclonal antibody conjugate is administered at a dose of 200 mg intravenously, delivered over one hour in a solution of 500 mL of normal saline with 5% human serum albumin. The patient is given a test dose of 1 mg intravenously initially, and during the infusion monitored for signs of hypersensitivity reactions.

The administration of the antibody is followed by the infusion of lymphokine, which in this case is Interleukin-2, delivered continuously at 3 x $10^6$ units per square meter per day for five consecutive days. After this infusion is begun, activated effector cells are administered on a daily basis for the five-day duration. The cells leukapheresed and placed in culture on Day 1 are infused first, and each day the next sample is delivered in the same sequence that they were harvested. The infusion of the cells initially begin slowly but, assuming there are no reactions, is completed in 20-40 minutes. Supportive care includes premedication of the patients prior to therapy with agents such as acetaminophen or indomethacin for fever, meperidine for chills, and other symptomatic measures such as anti-emetics and antihistamines.

The antibody/hapten administration is repeated at the same dose on Day 3 of therapy. This again precedes the administration of the effector cell infusion. The continuous infusion of IL-2 need not be interrupted for the antibody administration.

After the five days of therapy are completed, the patient has a rest period for five to 10 days, after which the therapy sequence is repeated. At the end of therapy, subcutaneous nodules are biopsied to evaluate the localization of antibody and the cellular response accompanying it. These observations may have a predictor value for tumor response. Patients are later followed to assess objective reduction in tumor size. Responders receive repeated courses of therapy, as long as the antiglobulin responses are not prohibitive. Measures to suppress an antiglobulin response may be employed in these patients (such as alkyllating agents, steroids, other immunologic manipulations).

### Example 1b:

Same as in Example 1a, except that naked antibody is used (instead of antibody with benzoate groups attached) for all procedures (in vitro and in vivo).

## Example 1c:

Same as in Example 1a, except that effector cells are not obtained by cytapheresis, but harvested from the bone marrow, as follows:

The patient is placed under anesthesia and multiple bone marrow aspirations are performed over the posterior iliac crest. 400-800 cc of bone marrow is collected, and serial cell counts are performed as the sample is collected, to ensure a yield of 3-5 x $10^{10}$ nucleated cells at the end of the harvest procedure. Cells are filtered through a stainless steel mesh to exclude spicules of bone. The resulting bone marrow suspension is then cultured in roller bottles, similar to the technique described in Example 1a for pheresed cells.

Treatment schedule also differs in this example from Example 1a. The bone marrow cells are cultured for 3-5 days, after which therapy begins. After the immunoconjugate and IL-2 are adminstered, 30-50% of the available cells are infused into the patient, as described in Example 1a. The remaining cells are maintained in culture and the treatment is repeated identically every 3-5 days, for up to three weeks. At that point response is assessed and further treatment decisions made.

## Example 2a:

In this example, pokeweed antiviral protein, which is a mitogen and potent antigen, will be linked with a monoclonal antibody against a melanoma-associated antigen. This complex will be used as the "engineered tumor antigen."

### The antibody:

The same antibody as in Example 1a is used.

### The Conjugation process:

Pokeweed antiviral protein (PAP) is reacted with 10 equivalents of SMCC (succinimidyl 4-(N-maleimidomethyl cyclohexane-1-carboxylate) by adding a DMSO solution (dimethyl sulfoxide) of SMCC (4 mg/mL) to protein in phosphate buffer (0.01 M, pH = 8.5). After 30 minutes at room temperature the protein is purified by size exclusion, by use of a column (such as the BioRad PD-10). To a solution of antibody a concentration of 2-5 mg/mL in 0.01 M phosphate buffer (pH = 7.5), is added sufficient DTT (dithiothreitol) to arrive at a final concentration of 25 mM with respect to DTT. The reaction mixture is kept at room temperature for 30 minutes to generate free sulhydryl groups on the antibody. DTT is removed by a size exclusion chromatography. DTT-treated antibody and SMCC-treated PAP are then mixed immediately and allowed to react for one hour. The conjugate is concentrated to about 500 microliters and injected through a sepharose-12 size exclusion column. The conjugate is eluted with pH 7.5 FPLC buffer (0.01 M phosphate, 0.5 M NaCl) and the fraction corresponding to the antibody-PAP conjugate is collected for use.

### Patient Selection

Same as in Example 1a.

### The Harvesting and Generation of Immune Effector Cells:

Same as in Example 1a.

**The Administration of Therapy:**

Same as in Example 1a.

**Example 2b:**

Same as in Example 2a, except that the in vitro immunization is performed with PAP alone (not the antibody conjugate). However, the patient is administered the antibody conjugate, and the remainder of the treatment is carried out as outlined in Example 2a (and 1a).

**Example 3a:**

In this example, a glycosylated monoclonal antibody is used, produced by recombinant yeast cells after the insertion of the relevant genes. Since these antibodies contain human structures resulting in increased antigenicity, no additional haptens, antigens or mitogens are necessary for use. All other aspects of the procedure are as described in Examples 1a and 2a.

**Example 4:**

In this example, a Class I MHC antigen, HLA-A2, or alloantigenic fragments from its heavy chain, are conjugated to an anti-tumor antibody. This immunoconjugate may be useful for treatment non-HLA-A2 recipient's tumors. Since cytotoxic T cells have been described which react to either the alpha-1 or alpha-2 domains of HLA-A2, the following preparations can be examined for efficacy in directing either existing of ex vivo expanded cytotoxic T cells to the tumor.

1. Isolated, solubilized HLA-A2.

This protein can be isolated and solubilized by the method of Pober et al. (Biochemistry 20,5625-32, 1981). It can then be cross-linked to an anti-tumor whole 1gG, (Fab′)$_2$ or Fa′b fragment using bifunctional crosslinking reagents as described in the preceding examples or by Pierce Chemical Co. (catalog, 1988, pp. 221-250) The immunoconjugate can be tested for anti-tumor acitivity.

2. Fragments of human leukocyte antigen-A2 (HLA-A2).

Fragments comprising the cytotoxic T-lymphocyte (CTL) binding site (alloantigen site), which has not been precisely defined for any MHC antigen, can be generated by synthesis of overlapping long peptide fragments from the known sequence of the antigen (Figueroa and Klein, 1986). Overlapping heavy chain peptides 40-residues long or longer, spaced 10-20 residues apart in the primary structure of the alpha-1 or alpha-2 domains, can be synthesized according to the methodology of Merrifield et al., Biochemistry 21, 5020-31, 1982, or Houghten, PNAS 82, 5131-35, 1985, or on a commercially available peptide synthesizer. The peptides can be cleaved from the solid phase resin and deprotected according to Tam et al., JACS 105, 6442-55, 1983, extracted with 20% acetic and, lyophilized and purified by reversed phase HPLC on a Vydac C-4 analytical column using a linear gradient of 100% water plus 0.1% triftworacetic acid (TFA) to 100% acetonitrile plus 0.1% TFA in 50 minutes. The peptide is analyzed using PTC-amino acid analysis (Heinrikson et al., Anal. Biochem. 136, 65-74, 1984). For HLA-A2, at least one synthetic peptide would include residues 98-113 of the heavy (alpha) chain, which has been shown to block the interaction of CTL's with HLA-A2 (Parham et al, 1987).

The features disclosed in the foregoing description, in the claims and/or in the accompanying drawings, may, both, separately and in any combination thereof, be material for realising the invention in diverse forms thereof.

## Claims

1. A composition comprising sensitized effector cells, and an immunogenic molecule comprising an antibody or a conjugate thereof that is capable of binding to selected target cells, wherein the immunogenic molecule is recognized by the sensitized effector cells, for use within a method for eradicating said selected target cells within a mammalian host, said sensitized effector cells being autologous to said host.

2. The composition of claim 1, further comprising an effector cell growth factor.

3. A kit for eradicating selected target cells within a mammalian host, comprising two separate compositions, one of which comprises sensitized effector cells which are autologous to said host, the other composition comprising an immunogenic molecule comprising an antibody or a conjugate thereof that is capable of binding to said target cells, wherein the immunogenic molecule is recognized by said sensitized effector cells.

4. The kit of claim 4, further including a third separate composition comprising an effector cell growth factor.

5. The composition or kit of claims 2 or 4 wherein the effector cell growth factor is a lymphokine.

6. The composition or kit of claim 5 wherein said lymphokine is IL-2.

7. The composition or kit of claims 1 or 3 wherein the immunogenic molecule is an immunoconjugate comprising an immunogenic polypeptide bound to a monoclonal antibody which binds to the target cells.

8. The composition or kit of claim 7 wherein the sensitized effector cells recognize the polypeptide.

9. The composition or kit of claims 7 or 8 wherein said polypeptide is an antigen selected from the group consisting of non-self human Class I or Class II histocompatibility antigens, non-human Class I or Class II histocompatibility antigens, and alloantigen domains thereof.

10. The composition or kit of claims 7 or 8 wherein said polypeptide is chosen from the group consisting of peptide T-cell epitopes, cell surface antigens derived from bacteria, viral coat proteins, and immunogenic fragments thereof.

11. The composition or kit of claims 1 or 3 wherein the target cells are cancer cells.

12. The composition or kit of claims 1 or 3 wherein said sensitized effector cells are sensitized cytotoxic T-cells.

13. An immunoconjugate comprising an antibody specific for target cells to be eradicated within a human or mammalian host, attached to a polypeptide, wherein the polypeptide is an antigen selected from the group consisting of non-self Class I or Class II histocompatibility antigens, non-human Class I or Class II histocompatibility antigens, and alloantigenic domains of the histocompatibility antigens.

14. An immunoconjugate comprising an antibody specific for target cells to be eradicated within a human or mammalian host, attached to a polypeptide, wherein the polypeptide is selected from the group consisting of peptide T-cell epitopes, cell surface antigens derived from bacteria, viral coat proteins, and immunogenic fragments thereof.

15. The immunoconjugate of claims 13 or 14 wherein the target cells are cancer cells and the antibody is a monoclonal antibody or a monoclonal antibody fragment specific for the cancer cells.

European Patent Office

# EUROPEAN SEARCH REPORT

Application number EP 89105058.5

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| A | CHEMICAL ABSTRACTS, vol. 104, no. 25, June 23, 1986, Columbus, Ohio, USA<br>S.SHU et al. "In vitro sensitization and expansion with viable tumor cells and interleukin 2 in the generation of specific therapeutic effector cells." page 447, abstract-no. 223 015a<br>& J. Immunol. 1986, 136(10), 3891-8<br>-- | 1,15 | A 61 K 39/395 |
| A | DD - A5 - 221 917 (OTSUKA PHARMACEUTICAL CO.)<br>+ Abstract +<br>-- | 1,15 | |
| P,A | GB - A - 2 197 323 (NATIONAL RESEARCH DEVELOPEMENT CORPORATION)<br>+ Abstract +<br>-- | 1,7,15 | TECHNICAL FIELDS SEARCHED (Int Cl 4)<br>A 61 K 39/00 |
| P,A | GB - A - 2 197 322 (NATIONAL RESEARCH DEVELOPEMENT CORPORATION)<br>+ Abstract +<br>-- | 1,7,14, 15 | |
| P,A | EP - A2 - 0 265 847 (OLYMPUS OPTICAL CO., LTD.)<br>+ Abstract +<br>---- | 1,5,6 | |

The present search report has been drawn up for all claims

| Place of search | Date of completion of the search | Examiner |
|---|---|---|
| VIENNA | 23-06-1989 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document